# EUROPEAN PATENT APPLICATION

(11) **EP 4 772 142 A1**
(43) Date of publication of application: **08.07.2026**
(21) Application number: 24869900.1
(22) Date of filing: 26.06.2024
(51) Int. Cl.: A61F 2/07, A61F 2/82, A61B 17/12

(54) **MEDICAL STENT SYSTEM**

(30) Priority: 25.09.2023 CN 202311250245
(71) Applicant: Shanghai MicroPort Endovascular MedTech (Group) Co., Ltd., Shanghai 201318 (CN)
(72) Inventor: ZHA, Cheng, Shanghai 201318 (CN); ZHANG, Wei, Shanghai 201318 (CN); ZHU, Yongfeng, Shanghai 201318 (CN); ZHU, Qing, Shanghai 201318 (CN); YUAN, Zhenyu, Shanghai 201318 (CN); XING, Zhikai, Shanghai 201318 (CN)
(74) Representative: Patentship Patentanwaltsgesellschaft mbH
(86) International application number: PCT/CN2024/101516
(87) International publication number: WO 2025/066340

(57) **Abstract**

The present invention provides a medical stent system including a stent graft and procoagulant members disposed on the outer circumferential surface of the stent graft. The procoagulant member includes a plurality of procoagulant fibers and are configured to fill an aneurysm sac to slow down a flow rate of blood entering the aneurysm sac, thereby facilitating formation of a blood clot in the aneurysm sac. With this arrangement, the occurrence of a Type II endoleak can be reduced or even prevented, avoiding continuous expansion of the aneurysm sac.

## Description

### TECHNICAL FIELD

The present invention pertains to the field of medical devices, and more particularly relates to a medical stent system.

### BACKGROUND

In recent years, interventional physicians have increasingly chosen endovascular aneurysm repair (EVAR) as the first-choice therapy for abdominal aortic aneurysms (AAAs). EVAR is a minimally invasive surgical procedure conducted through very small incisions, providing surgical opportunities for many high-risk patients who cannot tolerate open chest surgery.

However, data collected from clinical studies over a long period of time suggests that the efficacy of EVAR is not as good as expected. Studies show that, although EVAR exhibits a higher patient survival rate than open chest surgery 6 months after surgery, this advantage diminished over time - 8 years after surgery, overall survival rate of patients who have undergone open chest surgery is higher than the survival rate of patients who have undergone EVAR. This is possibly attributable to aortic aneurysm rupture secondary to EVAR. Endoleaks tend to occur following EVAR - statistics reveal that, the overall incidence of endoleaks following EVAR ranges from 20% to 50%, with the incidence of Type II endoleaks being particularly high, at 10% to 25%. A Type II endoleak may lead to an increasingly expansion of the aneurysm sac, which may end up with rupture.

### SUMMARY OF THE INVENTION

It is an object of the present invention to provide a medical stent system, which is designed to fill an aneurysm sac and slow down a flow rate of blood entering the aneurysm sac, facilitating clotting of blood in the aneurysm sac and preventing the occurrence of a Type II endoleak.

To this end, the present invention provides a medical stent system comprising a stent graft and procoagulant members disposed on the outer circumferential surface of the stent graft, wherein the procoagulant member comprises a plurality of procoagulant fibers and are configured to fill an aneurysm sac to slow down a flow rate of blood entering the aneurysm sac, thereby facilitating the formation of a blood clot in the aneurysm sac.

Optionally, each procoagulant member may further comprise a self-expanding shaping portion, wherein each procoagulant fiber is attached to the shaping portion, wherein the self-expanding shaping portion is coupled to the stent graft at one end and is free at the other end, and wherein at least part of the shaping portion is pre-shaped into a three-dimensional structure.

Optionally, the procoagulant members may be configured as a number of procoagulant assemblies, and wherein each assembly comprises a plurality of procoagulant members, wherein the procoagulant members are arranged at intervals along a circumference of the stent graft, and wherein the procoagulant members in each procoagulant assembly are arranged at intervals along an axis of the stent graft and are arranged helically about an axis of the stent graft.

Optionally, the stent graft may comprise a first sub-stent and two second sub-stents, wherein the two second sub-stents are coupled to a single axial end of the first sub-stent in parallel and communicate with the first sub-stent, wherein a portion of each second sub-stent comprises an embolization section, and wherein the procoagulant members are provided on the circumferential surface of the first sub-stent and on the circumferential surface of each embolization section.

Optionally, the procoagulant members provided on the first sub-stent may be configured as a number of first procoagulant assemblies, wherein each assembly comprises a plurality of procoagulant members, wherein the first procoagulant assemblies are arranged at intervals along a circumference of the first sub-stent, and wherein the procoagulant members in each first procoagulant assembly are arranged at intervals along an axis of the first sub-stent and are arranged helically about an axis of the first sub-stent; and/or wherein the procoagulant members on each embolization section are configured as a number of second procoagulant assemblies, wherein each assembly comprises a plurality of procoagulant members, wherein the second procoagulant assemblies on each embolization section are arranged at intervals along a circumference of a corresponding second sub-stent, and wherein the procoagulant members in each second procoagulant assembly are arranged at intervals along an axis of a corresponding second sub-stent and are arranged helically about an axis of the corresponding second sub-stent.

Optionally, the first sub-stent may comprise a plurality of stent rings arranged at intervals along an axis thereof, and wherein adjacent procoagulant members in each first procoagulant assembly are spaced apart by one to three of the stent rings.

Additionally or alternatively, each second sub-stent may comprise a plurality of stent rings spaced apart along its axis, wherein adjacent procoagulant members in each second procoagulant assembly are spaced apart by one to three of the stent rings.

Optionally, the procoagulant member provided on the outer circumferential surface of the first sub-stent may be inclined relative to an axis of the first sub-stent. Additionally or alternatively, the procoagulant member provided on the outer circumferential surface of the embolization section may be inclined relative to an axis of the respective second sub-stent.

Optionally, the first sub-stent may comprise a first section and a second section, which are coupled to each other along its axis, the second section brought at an end thereof away from the first section into communication with the two second sub-stents,
wherein the procoagulant fibers in each procoagulant member on the first section have a length of 5 mm to 50 mm, and the procoagulant fibers in each procoagulant member on the second section have a length of 50 mm to 110 mm; and
the procoagulant fibers in each procoagulant member on the second sub-stent have a length of 5 mm to 50 mm.

Additionally or alternatively, the number of procoagulant fibers in each procoagulant member on the first section may be smaller than the number of procoagulant fibers in each procoagulant member on the second section, and wherein the number of procoagulant fibers in each procoagulant member on the second section is greater than that in each of the procoagulant members on the second sub-stents.

Optionally, the stent graft may comprise a first sub-stent and two second sub-stents, wherein the two second sub-stents are coupled to a single axial end of the first sub-stent in parallel and communicate with the first sub-stent, wherein the first sub-stent comprises a first section and a second section that are coupled to each other along an axis thereof, wherein an end of the second section away from the first section is brought into communication with the two second sub-stents, wherein a portion of each second sub-stent comprises an embolization section, and wherein the procoagulant members are provided on the outer circumferential surface of the first sub-stent and on the outer circumferential surface of each embolization section, and
wherein: the shaping portion in the procoagulant member on the first section has an axial length of 5 mm to 20 mm; the shaping portion in the procoagulant member provided on the second section has an axial length of 15 mm to 50 mm; and/or the shaping portion on each embolization section has an axial length of 5 mm to 20 mm.

Optionally, a procoagulant coating layer may be provided on the exterior of at least some of the procoagulant members.

The inventive medical stent system offers the following advantages over the prior art:
It includes a stent graft and procoagulant members disposed on the outer circumferential surface of the stent graft. The procoagulant member includes a plurality of procoagulant fibers and is configured to fill an aneurysm sac to slow down a flow rate of blood entering the aneurysm sac, thereby facilitating the formation of a blood clot in the aneurysm sac. With this arrangement, the occurrence of a Type II endoleak can be reduced or even prevented, avoiding continuous expansion of the aneurysm sac.

Additionally, each procoagulant member may include a self-expanding shaping portion, wherein each procoagulant fiber is attached to the shaping portion, wherein the self-expanding shaping portion is coupled to the stent graft at one end and is free at the other end, and wherein at least a part of the shaping portion is pre-shaped into a three-dimensional structure. After the medical stent system is implanted into the aorta and at least part thereof is located in the aortic aneurysm sac, the shaping portion self-expands into the pre-shaped structure, concurrently with the procoagulant fibers thereon stretching freely. Supported by the shaping portion, the procoagulant fibers will not be collapsed onto the outer circumferential surface of the stent graft under the action of blood flow. Thus, the procoagulant fibers can effectively fill the aneurysm sac, effectively slowing down flow rate of blood entering the aneurysm sac. This facilitates the formation of a blood clot in the aneurysm sac, which blocks blood flow therein. As a result, there will be a reduced pressure in the aneurysm sac, avoiding the occurrence of a Type II endoleak.

Further, the procoagulant members may be configured as a number of procoagulant assemblies each comprising a plurality of procoagulant members. The procoagulant members may be arranged at intervals along a circumference of the stent graft, and wherein the procoagulant members in each procoagulant assembly are arranged at intervals along an axis of the stent graft and are arranged helically about an axis of the stent graft. Alternatively, the stent graft may include a first sub-stent and second sub-stents, and a part of each second sub-stent may comprise an embolization section. The procoagulant members may be provided on the first sub-stent and the embolization section of each second sub-stent. The procoagulant members provided on the first sub-stent may be configured as a number of first procoagulant assemblies each comprising a plurality of the procoagulant members. The first procoagulant assemblies may be arranged at intervals along a circumference of the first sub-stent, and the procoagulant members in each first procoagulant assembly may be arranged at intervals along an axis of the first sub-stent and are arranged helically about an axis of the first sub-stent. Additionally, the procoagulant members provided on each embolization section may be configured as a number of second procoagulant assemblies each comprising a plurality of procoagulant members. The second procoagulant assemblies provided on each embolization section may be arranged at intervals along a circumference of a corresponding second sub-stent, and wherein the procoagulant members in each second procoagulant assembly are arranged at intervals along an axis of a corresponding second sub-stent and are arranged helically about an axis of the corresponding second sub-stent. Such an arrangement of the procoagulant members enables effectively reduced overlaps between them, avoids entanglement of procoagulant members and allows them to fill the aneurysm sac, thereby slowing down the flow rate of the blood entering the aneurysm sac and facilitating the formation of a blood clot therein. Thus, the occurrence of a Type II endoleak can be prevented. Further, it effectively reduces the resistance that the medical stent system encounters when crimped into a delivery system during fabrication and when released into a blood vessel during use. This enables more robust fabrication and higher surgical safety.

### BRIEF DESCRIPTION OF THE DRAWINGS

The accompanying drawings are provided to facilitate a better understanding of the present invention and do not unduly limit the scope thereof in any sense, in which:
Fig. 1 depicts a schematic representation of a medical stent system according to an embodiment of the present invention, showing a first sub-stent and portions of two second sub-stents;
Fig. 2 shows a schematic representation of an abdominal aortic aneurysm (AAA);
Fig. 3 schematically illustrates the use of a medical stent system according to an embodiment of the present invention for therapeutic treatment of an AAA;
Fig. 4 depicts a schematic representation of a medical stent system according to an embodiment of the present invention and differs from Fig. 1 in a different procoagulant member arrangement;
Fig. 5 depicts a schematic representation of the procoagulant member in the medical stent system of Fig. 4;
Fig. 6 depicts a schematic representation of a shaping portion of a procoagulant member in the medical stent system of Fig. 4; and
Fig. 7 schematically illustrates the use of the medical stent system of Fig. 4 for therapeutic treatment of an AAA.

### List of Reference Numerals

110 first sub-stent; 111 first section; 112 second section; 120 second sub-stent; 121 embolization section; 200 procoagulant member; 210 procoagulant fiber; 220 shaping portion; 221 three-dimensional helix structure; 300 anchoring mechanism; 101 stent ring; 102 graft; 10 aortic aneurysm; 21 inflow tract; 22 outflow tract; 23 abdominal aorta; 24 left iliac artery; 25 right iliac artery.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mount", "couple", "connect" and all grammatical variations thereof should be interpreted in a broad sense. For example, a connection may be a permanent, detachable or integral connection, or a mechanical or electrical connection, or a direct or indirect connection with one or more intervening media, or an internal communication or interaction between two elements. Relational terms such as "first," "second," and the like may be used herein solely to distinguish one entity or action from another entity or action, without necessarily requiring or implying any actual such relationship or order between such entities or actions, or denoting or implying relative importance of them, or implicitly indicating the number of them. It is noted that the terms "central", "longitudinal", "transverse", "length" , "width", "thickness", "upper" , "lower", "front" , "rear", "left" , "right", "vertical" , "horizontal", "top" , "bottom", "inner" , "outer", "axial", "radial", "circumferential" and so on may be used herein to describe orientations or positional relationships as viewed in the annexed figures. They are for convenience and ease of description of the present invention only and do not indicate or imply that the described apparatus or element must comprise, or be constructed or operated in, a particular orientation. Therefore, they are not to be construed as limiting the present invention. Those of ordinary skill in the art can understand the specific meanings of the above-mentioned terms herein, depending on their context.

The present invention aims to provide a medical stent system, which can be used for therapeutic intervention of an aortic aneurysm by promoting embolization within its sac and thereby preventing the occurrence of a Type II endoleak therein, thereby further preventing increasing expansion and even rupture of the aneurysm sac caused by the Type II endoleak.

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. Throughout these drawings, the same numerals indicate the same elements.

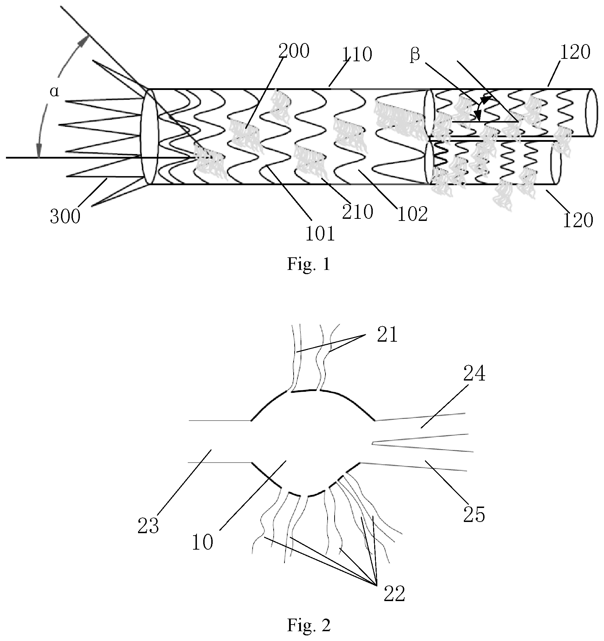

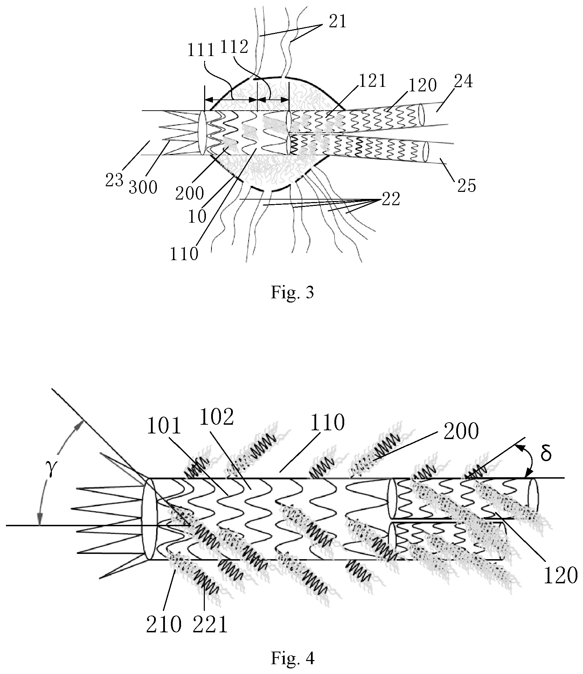

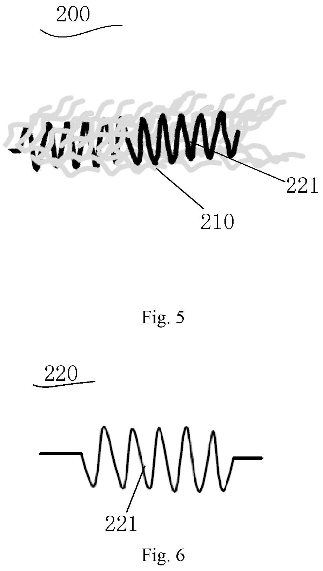

Referring to Figs. 1 and 4 to 6, embodiments disclosed herein provide a medical stent system including a stent graft and procoagulant members 200 disposed on an outer circumferential surface of the stent graft. The procoagulant members 200 include a plurality of procoagulant fibers 210. The medical stent system is used for therapeutic treatment of an aortic aneurysm 10, as shown in Fig. 2, with a plurality of branch vessels being in communication with a sac of the aortic aneurysm 10. Some of the branch vessels form inflow tracts 21, and the remaining ones form outflow tracts 22. During use of the medical stent system, as shown in Figs. 3 to 7, at least part of the stent graft is deployed within the sac of the aortic aneurysm 10 so that the procoagulant members 200 are located between an aneurysm wall and the outer circumferential surface of the stent graft. With this arrangement, the procoagulant fibers 210 of the procoagulant members 200 can slow down blood flow rate from the inflow tracts 21 into the aneurysm sac, facilitating the clotting of blood, i.e., the formation of a blood clot. The blood clot forms a space-occupying effect. The blood clot completely fills the interior of the aneurysm sac and blocks blood flow within the aneurysm sac. As a result, the occurrence of a Type II endoleak will be reduced or even prevented, avoiding the aortic aneurysm from experiencing a continuous expansion. Preferably, the procoagulant fibers 210 are cilia capable of promoting blood clot formation.

It will be understood that the medical stent system also includes an anchoring mechanism 300 coupled to one end of the stent graft. The medical stent system can be anchored within a blood vessel by the anchoring mechanism 300.

There may be branching arteries in the vicinity of the aortic aneurysm 10. For example, with continued reference to Fig. 2, the aortic aneurysm 10 may be located in the abdominal aorta 23, close to its bifurcation point with the left and right iliac arteries 24,25. In view of this, the medical stent system preferably includes one first sub-stent 110 and two second sub-stents 120. The two second sub-stents 120 are coupled to the same axial end of the first sub-stent 110 in parallel. In particular, the anchoring mechanism 300 may be provided at one end of the first sub-stent 110, and the two second sub-stents 120 may be coupled to the first sub-stent 110 at the end further away from the anchoring mechanism 300. The second sub-stents 120 may be both brought into communication with the first sub-stent 110. During use, one of the second sub-stents 120 is at least partially inserted into the left iliac artery 24, and the other second sub-stent 120 is at least partially inserted into the right iliac artery 25.

When each of the second sub-stents 120 has a section located within the aortic aneurysm 10, this section may be referred to as an embolization section 121. Thus, the embolization sections 121 are coupled to the first sub-stent 110. For this reason, the procoagulant members 200 may be provided on outer circumferential surfaces of the first sub-stent 110 and each embolization section 121.

In the present embodiment, the procoagulant members 200 provided on the first sub-stent 110 are configured as a number of first procoagulant assemblies (not labeled), and the procoagulant members 200 provided on each embolization section 121 are configured as a number of second procoagulant assemblies (not labeled). In other words, the first sub-stent 110 is provided thereon with a number of first procoagulant assemblies, and each embolization section 121 is provided thereon with a number of second procoagulant assemblies. Each first procoagulant assembly may include a plurality of procoagulant members 200, and each second procoagulant assembly may also include a plurality of procoagulant members 200.

It will be understood that, if there are no branching arteries in the vicinity of the aortic aneurysm 10, the stent graft may include only the first sub-stent, and the second sub-stents may be omitted (this case is not shown). Accordingly, the stent graft may be provided simply as a straight, cylindrical stent.

Optional configurations for the medical stent are described below in the context of it including the first sub-stent 110, the second sub-stents 120, and the first and second procoagulant assemblies, as an example.

Optionally, if there is a plurality of first procoagulant assemblies, they may be arranged at intervals along the circumference of the first sub-stent 110. All the procoagulant members 200 in each first procoagulant assembly may be arranged at intervals along the axis of the first sub-stent 110, and may be arranged helically about an axis of the first sub-stent 110. Likewise, each embolization section 121 may be provided thereon with at least one second procoagulant assembly. If there is a plurality of second procoagulant assemblies provided on each embolization section 121, all the second procoagulant assemblies on each embolization section 121 may be arranged at intervals along circumference of corresponding second sub-stent 120. Moreover, all the procoagulant members 200 in each second procoagulant assembly may be arranged at intervals along the axis of the first sub-stent 110, and may be arranged helically about an axis of the corresponding second sub-stent 120.

Such an arrangement of the first procoagulant assembly on the first sub-stent 110 and the second procoagulant assembly on the individual second sub-stents enables reduced overlaps between the procoagulant members 200, avoids entanglement of procoagulant members and allows them to fill an aneurysm sac to slow down blood flow into the aneurysm sac, facilitating blood clotting within the aneurysm sac and preventing the occurrence of a Type II endoleak. Further, it allows the medical stent system to be crimped to a smaller radial dimension, resulting in reduced resistance from a delivery system that the medical stent system encounters when crimped into the delivery system during fabrication and when released into a blood vessel during use. This enables more robust fabrication and higher surgical safety.

The first sub-stent 110 is substantially of the same structure as the second sub-stents 120. Specifically, each stent graft (i.e., each of the first sub-stent 110 and the second sub-stents 120) may include multiple stent rings 101 and a graft 102. In each stent graft (i.e., in each of the first sub-stent 110 and the second sub-stents 120), the stent rings 101 may be spaced apart along an axis of the specific stent graft, and the graft 102 may be attached to all the stent rings 101. In some embodiments, the graft 102 includes at least an outer graft sewn to outer circumferential surfaces of the stent rings 101.

In the first sub-stent 110, adjacent procoagulant members 200 within each single first procoagulant assembly are preferably spaced by one to three of the stent rings 101, in order to allow them to have further reduced overlaps. Similarly, in each second sub-stent 120, adjacent procoagulant members 200 within each single second procoagulant assembly are preferably spaced by one to three of the stent rings 101.

In some embodiments, the procoagulant fibers 210 in the procoagulant members 200 are directly attached to the stent graft 100, and the procoagulant fibers 210 in each single procoagulant member 200 are arranged in a direction forming an angle with an axis of the component stent. That is, the procoagulant fibers 210 in each single one of the procoagulant members 200 on the outer circumferential surface of the first sub-stent 110 are arranged in a direction inclined at an angle relative to the axis of the first sub-stent 110, and the procoagulant fibers 210 in each single one of the procoagulant members 200 on the outer circumferential surface of each embolization section are arranged in a direction inclined at an angle relative to the axis of the corresponding second sub-stent.

In particular, in the first procoagulant assembly, all the procoagulant fibers 210 in each single procoagulant member 200 may be arranged in a first predefined path, and a projection of the first predefined path may be inclined relative to a projection of the axis of the first sub-stent 110 on a plane parallel to the axis of the first sub-stent 110. Optionally, on the plane parallel to the axis of the first sub-stent 110, the projection of the first predefined path may form a third angle α in the range of from -45° to 45° with the projection of the axis of the first sub-stent 110. This arrangement allows the procoagulant fibers 210 in different procoagulant members 200 in the first procoagulant assembly to have reduced overlaps.

Moreover, in the second procoagulant assembly, all the procoagulant fibers 210 in each single procoagulant member 200 may be arranged in a second predefined path, and a projection of the second predefined path may be inclined relative to a projection of the axis of the second sub-stent 120 on a plane parallel to the axis of the second sub-stent 120. This allows adjacent procoagulant members 200 in each single second procoagulant assembly to have reduced overlaps. Optionally, on the plane parallel to the axis of the second sub-stent 120, the projection of the second predefined path may form a fourth angle β in the range of from -45° to 45° with the projection of the axis of the second sub-stent 120.

In each procoagulant member 200, the procoagulant fibers 210 may have a length depending on the location of the specific procoagulant member 200 in the aneurysm sac.

In detail, referring to Figs. 2 and 3, the aortic aneurysm 10 may have a diameter, which increases and then decreases along an axis of the abdominal aorta 23. The first sub-stent 110 includes a first section 111 and a second section 112, which are coupled to each other along its axis. An end of the first section 111 away from the second section 112 is coupled to the anchoring mechanism 300, and an end of the second section 112 away from the first section 111 is coupled to both second sub-stents 120. The second section 112 is configured to be located around the middle of the aortic aneurysm 10, and the first section 111 and the embolization sections 121 are configured to be located around opposite axial ends of the aortic aneurysm 10. That is, the second section 112 is configured to be located in a portion of the aneurysm sac with a relatively large diameter, while the first section 111 and the embolization sections 121 are configured to be located in portions of the aneurysm sac with relatively small diameters.

Accordingly, the length of the procoagulant fibers 210 in the procoagulant members 200 on the first section 111 may be less than the length of the procoagulant fibers 210 in the procoagulant members 200 on the second section 112. In addition, the length of the procoagulant fibers 210 in the procoagulant members 200 of the second procoagulant assembly may be less than the length of the procoagulant fibers 210 in the procoagulant members 200 on the second section 112. It will be understood that each procoagulant fiber 210 has two opposite ends, one of which is attached to the respective sub-stent (the first sub-stent 110 or one of the second sub-stents 120), and the other is a free end. The length of the procoagulant fiber 210 may be measured between its opposite ends when it is in the shape of a straight line. This arrangement can ensure that a blood clot formed in the aneurysm sac blocks blood flow into the aneurysm sac, while minimizing the use of material for the procoagulant fibers 210.

Optionally, the length of the procoagulant fibers 210 in the procoagulant members 200 on the first section 111 and the length of the procoagulant fibers 210 in the procoagulant members 200 of the second procoagulant assemblies may range from 5 mm to 50 mm, and the length of the procoagulant fibers 210 in the procoagulant members 200 on the second section 112 may range from 50 mm to 100 mm.

Additionally or alternatively, the number of procoagulant fibers 210 in the procoagulant members 200 on the second section 112 may be greater than the number of procoagulant fibers 210 in the procoagulant members 200 on the first section 111 and the number of procoagulant fibers 210 in the procoagulant members 200 of the second procoagulant assemblies. This can likewise ensure that a blood clot is formed in the aneurysm sac, while minimizing the use of material for the procoagulant fibers 210.

Optionally, each stent ring 101 includes a plurality of stent struts (not labeled), which are connected end to end. Adjacent stent struts comprise a V-shape.

In some embodiments, the procoagulant fibers 210 in the procoagulant members 200 of the first procoagulant assembly are sewn onto the first sub-stent 110 with sutures. In one preferred implementation, each procoagulant member 200 in the first procoagulant assemblies is provided on a stent ring 101, and the first predefined path of each procoagulant member 200 coincides with a stent strut. In addition, the procoagulant fibers 210 in the procoagulant members 200 are sewn onto the respective stent struts with the same sutures that sew the graft 102 to the stent rings 101. This arrangement can reduce the number of suture holes formed on the graft 102 of the first sub-stent 110 due to the stitching. It will be understood that this also allows projections of the stent struts in the first sub-stent 110 to form the third angle α with a projection of the axis of the first sub-stent 110 on a plane parallel to the axis of the first sub-stent 110.

Further, the procoagulant fibers 210 in the procoagulant members 200 of the second procoagulant assemblies are all sewn onto the second sub-stents 120 with sutures. Optionally, each procoagulant member 200 in the second procoagulant assembly may be provided on a stent ring 101, and the second predefined path of each procoagulant member 200 may coincide with a stent strut. In addition, the procoagulant fibers 210 in the procoagulant members 200 may be all sewn onto the respective stent struts with the same sutures that sew the grafts 102 to the stent rings 101. It will be understood that this allows projections of the stent struts in each second sub-stent 120 to form the fourth angle β with a projection of the axis of the second sub-stent 120 on a plane parallel to the axis of the second sub-stent 120.

Further, a procoagulant coating layer (not shown) may be provided on the exterior of the procoagulant fibers 210 in at least some of the procoagulant members 200. Preferably, the procoagulant coating layer is provided on the exterior of shaping portions 220 and the procoagulant fibers 210 in all the procoagulant members 200. The presence of the procoagulant coating layer can accelerate blood clotting, i.e., speeding up the formation of a blood clot within the aneurysm sac and filling of the aneurysm sac. The procoagulant coating layer may include hydrogel.

In alternative embodiments, referring to Figs. 4 to 6, each procoagulant member 200 may include a self-expanding shaping portion 220 and procoagulant fibers 210 attached to the exterior of the shaping portion 220. The shaping portion 220 is coupled to the stent graft 100 at one end and free at the other end. Moreover, at least part of the shaping portion 220 is pre-shaped into a three-dimensional structure. In a typical implementation, at least part of the shaping portion 220 is pre-shaped into a three-dimensional helix structure 221.

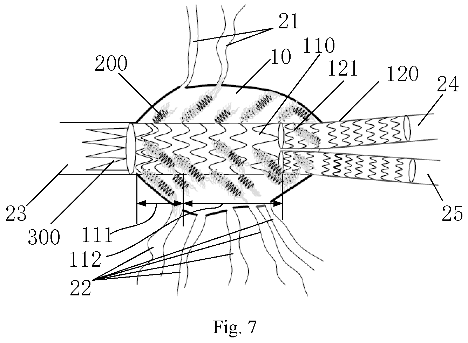

After the medical stent system is implanted into the blood vessel, as shown in Fig. 7, the self-expanding shaping portion 220 self-expands into a shape comprising the three-dimensional helix structure 221, simultaneously, the procoagulant fibers 210 freely stretching within the aneurysm sac with the expansion of the self-expanding shaping portion 220, without being collapsed onto the exterior of the stent grafts (i.e., the first sub-stent 110 and the second sub-stents 120) under the action of blood flow. Moreover, the shaping portions 220 themselves contribute to the filling of the aneurysm sac. With this configuration, the procoagulant members 200 can desirably facilitate blood clotting and speed up the formation of a blood clot in the aneurysm sac. Further, this configuration of the procoagulant fibers 210 can prevent them from kinking due to blood reflux. The shaping portions 220 may be made of a shape memory alloy, such as a nickel-titanium alloy, or another highly elastic material, and have been pre-shaped to comprise the three-dimensional helix structure 221.

It will be understood that, in these embodiments, the procoagulant members 200 may be arranged in the same way as described above. That is, all the first procoagulant assemblies may be arranged at intervals along the circumference of the first sub-stent 110, and all the procoagulant members 200 of each single first procoagulant assembly may be arranged at intervals along the axis of the first sub-stent 110, and may be arranged helically about the axis of the first sub-stent 110. Additionally, all the second procoagulant assemblies on each embolization section 121 may be arranged at intervals along the circumference of the respective second sub-stent 120, and all the procoagulant members 200 of each single second procoagulant assembly may be arranged along the axis of the second sub-stent 120, and may be arranged helically about the axis of the second sub-stent 120.

Further, the axis of the three-dimensional helix structure 221 in the first procoagulant assembly may be inclined with respect to the axis of the first sub-stent 110. In addition, in the second procoagulant assembly, the axis of the three-dimensional helix structure 221 of the shaping portions 220 may be inclined with respect to the axis of the specific second sub-stent 120.

In the first procoagulant assembly, the axis of the three-dimensional helix structure 221 in the procoagulant member 200 may form a first angle γ in the range of -45° to 45° with the axis of the first sub-stent 110. In the second procoagulant assembly, the axis of the three-dimensional helix structure 221 in the procoagulant member 200 may form a second angle δ in the range of -45° to 45° with the axis of the second sub-stent 120 that it is coupled to.

In these embodiments, the shaping portions 220 coupled to the second section 112 have an axial length greater than both an axial length of the shaping portions 220 coupled to the first section 111 and an axial length of the shaping portions 220 coupled to the second sub-stents 120. Here, the term "axial" of the shaping portions 220 refers to a direction along the axis of the three-dimensional helix structure 221 of the shaping portion 220.

Optionally, the axial length of the shaping portion 220 coupled to the first section 111 may range from 5 mm to 20 mm, and the axial length of the shaping portion 220 coupled to the second section 112 may range from 15 mm to 50 mm. The axial length of the shaping portion 220 coupled to the second sub-stents 120 may range from 5 mm to 20 mm. With this arrangement, the procoagulant members 200 at various locations in the aneurysm sac can fully fill the aneurysm sac, effectively slowing down blood flow rate entering the aneurysm sac and facilitating blood clotting therein. In these embodiments, a procoagulant coating layer (not shown) may be provided on the exterior of the shaping portions 220 and/or the procoagulant fibers 210 in at least some of the procoagulant members 200. Preferably, the procoagulant coating layer is provided on the exterior of the shaping portions 220 and the procoagulant fibers 210 in all the procoagulant members 200. The presence of the procoagulant coating layer can accelerate blood clotting, i.e., speeding up the formation of a blood clot within the aneurysm sac and the filling of the aneurysm sac. The procoagulant coating layer may include hydrogel.

In summary, embodiments disclosed herein provide a medical stent system including a stent graft provided with procoagulant members on its outer circumferential surface. When the medical stent system is used for therapeutic treatment of an aneurysm, the procoagulant members can slow down flow rate of blood entering the aneurysm sac, facilitating the formation of a blood clot to fill the aneurysm sac and thus reducing the occurrence of a Type II endoleak. In particular, each procoagulant member may include a shaping portion and procoagulant fibers attached thereto. The presence of the shaping portions can prevent the procoagulant fibers from being collapsed onto the stent graft. As a result, the procoagulant members can effectively fill the aneurysm sac and further facilitate blood clot formation therein.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical stent system, comprising a stent graft and procoagulant members disposed on an outer circumferential surface of the stent graft, wherein the procoagulant member comprises a plurality of procoagulant fibers and is configured to fill an aneurysm sac to slow down a flow rate of blood entering the aneurysm sac, thereby facilitating formation of a blood clot in the aneurysm sac.

2. The medical stent system according to claim 1, wherein the procoagulant member further comprises a self-expanding shaping portion, wherein each procoagulant fiber is attached to the shaping portion, wherein the shaping portion is coupled to the stent graft at one end and is free at the other end, and wherein at least a part of the shaping portion is pre-shaped into a three-dimensional structure.

3. The medical stent system according to claim 1 or 2, wherein the procoagulant members are configured as a number of procoagulant assemblies, and wherein each assembly comprises a plurality of procoagulant members,
wherein the procoagulant members are arranged at intervals along a circumference of the stent graft, and wherein the procoagulant members in each procoagulant assembly are arranged at intervals along an axis of the stent graft and are arranged helically about an axis of the stent graft.

4. The medical stent system according to claim 1 or 2, wherein the stent graft comprises a first sub-stent and two second sub-stents, wherein the two second sub-stents are coupled to a single axial end of the first sub-stent in parallel and communicate with the first sub-stent, wherein a portion of each second sub-stent comprises an embolization section, and wherein the procoagulant members are provided on an outer circumferential surface of the first sub-stent and on an outer circumferential surface of each embolization section.

5. The medical stent system according to claim 4, wherein the procoagulant members provided on the first sub-stent are configured as a number of first procoagulant assemblies, wherein each assembly comprises a plurality of procoagulant members, wherein the first procoagulant assemblies are arranged at intervals along a circumference of the first sub-stent, and wherein the procoagulant members in each first procoagulant assembly are arranged at intervals along an axis of the first sub-stent and are arranged helically about an axis of the first sub-stent.

6. The medical stent system according to claim 5, wherein the first sub-stent comprises a plurality of stent rings arranged at intervals along an axis thereof, and wherein adjacent procoagulant members in each first procoagulant assembly are spaced apart by one to three of the stent rings.

7. The medical stent system according to any one of claims 4 to 6, wherein the procoagulant members on each embolization section are configured as a number of second procoagulant assemblies, wherein each second procoagulant assembly comprises a plurality of procoagulant members, wherein the second procoagulant assemblies on each embolization section are arranged at intervals along a circumference of a corresponding second sub-stent, and wherein the procoagulant members in each second procoagulant assembly are arranged at intervals along an axis of a corresponding second sub-stent and are arranged helically about an axis of the corresponding second sub-stent.

8. The medical stent system according to claim 7, wherein each second sub-stent comprises a plurality of stent rings arranged at intervals along an axis thereof, wherein adjacent procoagulant members in each second procoagulant assembly are spaced apart by one to three of the stent rings.

9. The medical stent system according to claim 4, wherein the procoagulant member provided on the outer circumferential surface of the first sub-stent is inclined relative to an axis of the first sub-stent.

10. The medical stent system according to claim 4 or 9, wherein the procoagulant member provided on the outer circumferential surface of the embolization section is inclined relative to an axis of a corresponding second sub-stent.

11. The medical stent system according to claim 4, wherein the first sub-stent comprises a first section and a second section that are coupled to each other along an axis thereof, wherein an end of the second section away from the first section communicates with the two second sub-stents,
wherein the procoagulant fiber in each procoagulant member on the first section has a length of 5 mm to 50 mm, and wherein the procoagulant fiber in each procoagulant member on the second section has a length of 50 mm to 110 mm; and
wherein the procoagulant fiber in each procoagulant member on the second sub-stent has a length of 5 mm to 50 mm.

12. The medical stent system according to claim 4 or 11, wherein a number of procoagulant fibers in each procoagulant member on the first section is smaller than a number of procoagulant fibers in each procoagulant member on the second section, and wherein a number of procoagulant fibers in each procoagulant member on the second section is greater than a number of procoagulant fibers in each procoagulant member on the second sub-stent.

13. The medical stent system according to claim 2, wherein the stent graft comprises a first sub-stent and two second sub-stents, wherein the two second sub-stents are coupled to a single axial end of the first sub-stent in parallel and communicate with the first sub-stent, wherein the first sub-stent comprises a first section and a second section that are coupled to each other along an axis thereof, wherein an end of the second section away from the first section is brought into communication with the two second sub-stents, wherein a portion of each second sub-stent comprises an embolization section, and wherein the procoagulant members are provided on an outer circumferential surface of the first sub-stent and on an outer circumferential surface of each embolization section, and
wherein: the shaping portion in the procoagulant member on the first section has an axial length of 5 mm to 20 mm; the shaping portion in the procoagulant member on the second section has an axial length of 15 mm to 50 mm; and/or the shaping portion on each embolization section has an axial length of 5 mm to 20 mm.

14. The medical stent system according to claim 1 or 2, wherein a procoagulant coating layer is provided on an exterior of at least some of the procoagulant members.
